# EUROPEAN PATENT APPLICATION

(11) **EP 3 586 745 A1**
(43) Date of publication of application: **01.01.2020**
(21) Application number: 18213313.2
(22) Date of filing: 18.12.2018
(51) Int. Cl.: A61B 5/11, A61B 5/00

(54) **A SYSTEM FOR TRACKING HUMAN MOTION**

(30) Priority: 26.06.2018 PT 2018110804
(71) Applicant: Sword Health, S.A., 4000 437 Oporto (PT)
(72) Inventor: MARQUES GABRIEL, Ivo Emanuel, 4000-437 Porto (PT); FERRO VENTO, Virgílio António, 4000-437 Porto (PT)
(74) Representative: DTS Patent- und Rechtsanwälte Schnekenbühl und Partner mbB

(57) **Abstract**

The present invention relates to a system (10) for tracking human motion comprising
- at least one sensor unit (12, 14),
- at least one sensor holder (16, 18),
wherein the sensor unit (12, 14) comprises a sensor communication interface (24, 26) and wherein the sensor holder (16, 18) comprises a counter communication interface (28, 30), wherein the sensor unit (12, 14) is configured such that depending on the correct placement of the sensor unit (12, 14) to the sensor holder (16, 18) a communication link (L1, L2) with the sensor communication interface (24, 26) and the counter communication interface (28, 30) is established and it can be inferred by the sensor unit (12, 14), whether it is attached to the sensor holder (16, 18) or not.

Furthermore, the invention relates to a method for sensor unit placement verification for a system (10) for tracking human motion.

## Description

The present invention relates to a system for tracking human motion, for example a system for tracking human motion in connection with rehabilitation applications, and a method for sensor unit placement verification for a system for tracking human motion.

During rehabilitation, one of the challenges is to control rehabilitation exercises.

From EP 2 750 120 A1 an exercise information display system and exercise information display method is already known. The system includes a sensor device, which obtains data associated with a motion status of a human body during an exercise, a data processing device which generates plural types of exercise information based on the data obtained by the sensor device and a viewing device which displays from among the plural types of exorcise information at least first information indicating a posture of the human body during the exercise and second information associated with the first information in the display format, where the first information and a second information are displayed in connection with each other.

A system capable of tracking human motion requires the user to attach a set of sensors to a set of straps he is wearing. Each of those sensors must be placed on anatomical landmarks depending upon the limb whose motion is to be tracked. The same system also requires the user to attach the sensors to a sensor station for the purpose of charging them as well as performing an inspection thereof. As such, the following is required for using this system:
- Each sensor must be matched to the corresponding strap before the motion tracking procedure, as the system assumes an anatomical landmark for each sensor - this may be accomplished by using a color code that matches the sensor to the strap of the same color or, alternatively, by the means of calibration movements before usage.
- The sensors must be kept correctly attached to the strap throughout the motion tracking procedure.
- The sensors must be correctly attached to the sensor station for the charging and inspection procedures.

Due to these requirements and the fact that it is not clear for the user what he must do at a given point while using the system, the following problems arise:
- The user does not place the sensors in the straps because he does not understand he must do so.
- The user misplaces the sensors by having them incorrectly attached to the straps.
- The user misplaces the sensors by switching positions between two sensors since this match is cumbersome and prone to failure.
- The sensors may detach during the movement without the user realizing.
- The user does not place the sensors in the station because he does not understand he must do so.

These events result in a corruption of the motion tracking procedure, an invalid sensor inspection and a lack of battery of the sensors. In sum, using the system correctly requires great understanding of its requirements (which impairs the user experience) and failing to do so has severe consequences.

It is therefore an option of the present invention to provide a system for tracking human motion which provides an enhanced solution for correct sensor placement and avoiding misplacement of the sensors, but also a better solution for recharging of the sensors and the respective electronics.

This object is solved according to present invention by a system for tracking human motion with the features of claim 1.

Accordingly, a system for tracking human motion is provided, comprising
- at least one sensor unit,
- at least one sensor holder,
wherein the sensor unit comprises a sensor communication interface and wherein the sensor holder comprises a counter communication interface, wherein the sensor unit is configured such that depending on the correct placement of the sensor unit to the sensor holder a communication link with the sensor communication interface and the counter communication interface is established and it can be inferred by the sensor unit, whether it is attached to the sensor holder or not.

In particular, the sensor unit comprises at least one sensor.

The invention is based on the basic idea that by means of the sensor communication interface and the counter communication interface of the sensor holder a misplacement of the sensor and the holder can be identified by the system immediately. For example, each of the sensor holders may be equipped with a hardware interface that connects to a homologous interface in the sensor unit upon attachment. This interface in the connection enables and provides each individual sensor unit with the capability of inferring whether it is attached to the sensor holder or to which other system it is attached.

As a result, the system is able to make sure that the sensor unit is correctly attached to the sensor holder and will provide the respective information if this is not the case. So, the system for tracking human motion checks before but also during exercises and the motion tracking, whether the system unit is correctly attached or not.

All sensor units may be completely identical in their appearance and/or functionality.

They can be identical in terms of form and/or technical features, i.e. the used electronics inside the sensor units.

On the other hand, the sensor holder may have an individualization and/or identification element.

This individualization and/or identification element can be recognized and/or identified by the sensor unit.

So, each sensor always knows to which sensor holder it is attached and thus the system always is able to identify, which sensor is attached to which sensor holder and thus to which portion of the body.

Thus, it is possible that during use a strict correspondence between the sensor units and the sensor holders is established, although the sensor units are identical.

It is also not necessary, that the sensor units have a code (although it is generally possible to have a color coding or the like on a sensor unit).

Furthermore, it is possible that only the sensor holders have a coding and an identification of the limb, on which they should be placed and to which they belong.

Furthermore, also information by the system can be provided, when it is detected that the sensor unit with its at least one sensor is no longer correctly placed on the corresponding sensor holder, i.e. for example that a de-attachment of the sensor unit from the sensor holder is happening during motion tracking procedure. Also a sensor unit inspection procedure can be provided, and the information can be obtained, whether such a sensor inspection procedure was valid by requiring the user to have the sensor unit correctly attached to the corresponding sensor holder. With this, also the training of the user and the handling of the system by the user can be enhanced and simplified as the system is now able to guide the user through each step by providing relevant and adaptive feedback whilst ensuring proper functioning.

Furthermore, it is possible that the sensor holder is a wearable. Such a wearable may be attached to any limb or any other part of the body of the patient. So human motion tracking can be done in a very simple and effective way.

In particular, the sensor holder may be a wearable strap. Such a strap may have an elastic strap and sensor unit attachment element, wherein this element is attached to elastic bands or straps with which the sensor holder may be placed on the body or a limb of the patient.

Furthermore, the sensor unit may comprise at least one of an inertial sensor, gyroscope, acceleration sensor, piezo sensor, magnetic sensor, optical sensor or the like. The sensor unit shall comprise at least one sensor, with which the movement of a body part or a limb may be tracked and sensed. The sensing may be direct and/or indirect. Preferably, an inertial sensor may be used.

Additionally, the system may further comprise a main unit, wherein the main unit may be configured such that motion data as well as the information regarding the attachment of the sensor unit to the sensor holder can be tracked and/or processed. In other words, the main unit may process the motion data as well as the information regarding the attachment of each sensor unit and may provide feedback to the user. In particular, the main unit may be embodied by a tablet device with a suitable software tool installed on the tablet device. Such a software tool may be for example a so-called "App".

Moreover, the system may further comprise a sensor station, which is configured and arranged for re-charging of the sensor unit. The sensor station may be configured such that it may hold the at least one sensor unit for charging and inspection purposes. The sensor station may be in connection (wireless and/or wirebound) with the attachment communication interface, wherein this component may communicate with the main unit through a cable or a wireless communication protocol.

Additionally, the sensor station may comprise a plurality of receiving slots for more than one sensor unit. By this, also a plurality of sensor units can be re-charged and checked at the same time.

Furthermore, the system comprises a plurality of sensor units and/or a plurality of sensor holders. By providing a plurality of sensor units a more detailed tracking of the motion can be provided. Also more complex movements and motions of the person can be tracked.

Moreover, the counter communication interface may comprise at least one passive element. By this, the sensor holder can be designed as a complete passive element. So, no additional energy source is needed for the sensor holder. The re-charging requirement only applies to the sensor unit, but not to the sensor holder.

In particular, the passive element may be or may comprise a resistor. With this, a very reliable and easy design can be achieved.

Additionally, it is possible that the resistor has a specific resistance, which is corresponding to a specific sensor holder. By this, the resistor can be designed such that it only matches to one specific sensor holder, i.e. it forms a representative item for one specific sensor holder.

By this, the verification of correct attachment of the sensor unit and the respective sensor holder can be done.

Furthermore, it is possible that a plurality of sensor holders may be provided, and each sensor holder has its own specific resistor with its own specific resistance, thereby identifying the sensor holder.

Moreover, the present invention relates to a method for sensor unit placement verification for a system for tracking human motion. Accordingly, the method is performed with a system for tracking human motion, wherein the system comprises
- at least one sensor unit,
- at least one sensor holder,
wherein the method further comprises at least the following steps:
- the sensor unit is placed in the sensor holder;
- a communication link between the sensor unit and the sensor holder is established; and
- based on the communication link and the information exchanged via this communication link it is inferred by the sensor unit, whether it is attached to the sensor holder or not.

Also, it is possible that with the established communication link an individualized element of the sensor holder is checked and identified by means of the sensor unit.

The system used for performing the method may be the system for tracking human motion as described above.

Further details and advantages will be shown in the figures.

It is shown in
- Fig. 1: a perspective view of an embodiment of a system for tracking human motion according to the present invention;
- Fig. 2: a schematic overview of the communication between the sensor units and the main unit of the system according to Fig. 1;
- Fig. 3: a schematic view of the communication between the station and the main unit;
- Fig. 4: a flow chart of the sensor unit inspection procedures; and
- Fig. 5: a flow chart of the sensor unit charging procedure according to the present invention.

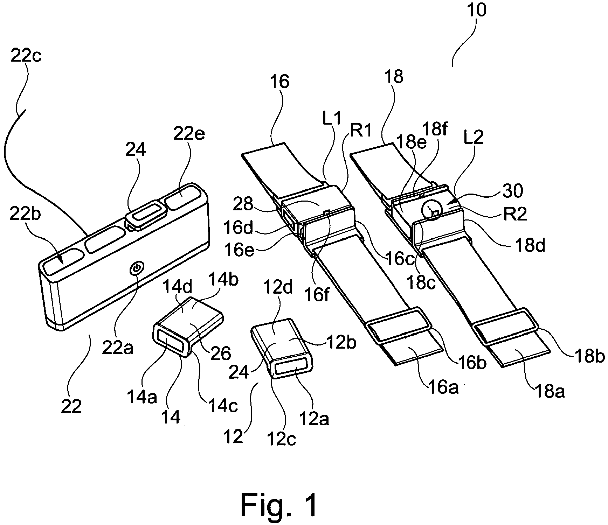

**Fig. 1** shows in a perspective view an embodiment of a system 10 for tracking human motion according to the present invention.

The system 10 comprises in the shown embodiment two sensor units 12 and 14.

Furthermore, there are sensor holders 16, 18, which are configured to receive and be attached with the sensor unit 12 or 14.

In the shown embodiment, sensor holders 16, 18 are configured and arranged to correspond and be attached during a work cycle/tracking cycle with sensor units 12, 14.

As long as sensor units 12, 14 are used, they can be received and placed in the sensor holders 16, 18, which form the respective counterelement.

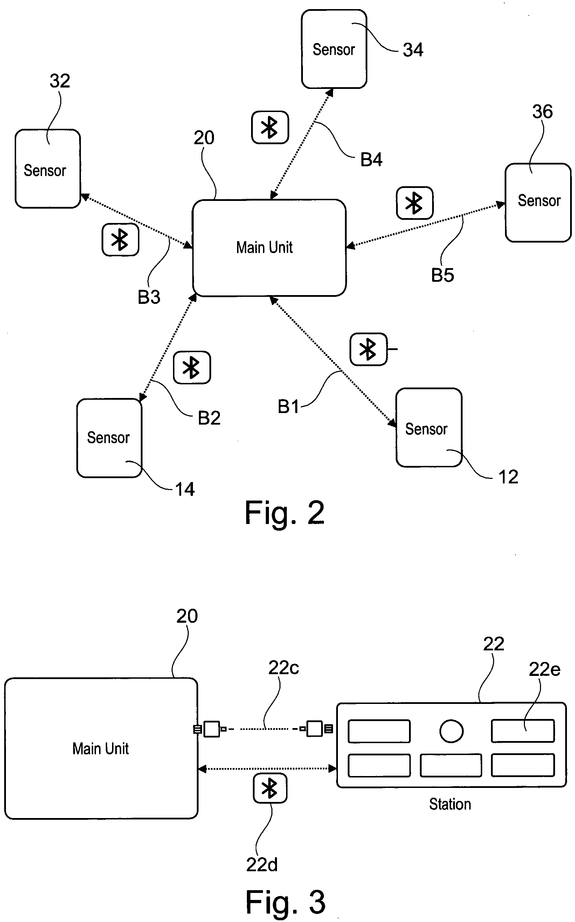

Moreover, the system 10 comprises a main unit 20, cf. also **Fig. 2** and **Fig. 3****.**

The main unit 20 is in the shown embodiment realized by a tablet PC.

Additionally, the system 10 comprises a sensor station 22.

The sensor station 22 comprises a plurality of receiving slots 22e, which are configured to receive the sensor units 12, 14.

The sensor station 22 is configured and arranged for re-charging of the sensor units 12, 14.

The sensor units 12, 14 comprise each a sensor communication interface 24, 26 and the sensor holders 16, 18 also comprise each a counter communication interface 28, 30.

The sensor units 12, 14 are identical. This applies in particular to the technical features and also to the design of the sensor units 12, 14 (and other sensor units).

Each sensor unit 12 or 14 has an L-form.

However, each sensor unit 12 or 14 is not personalized/individualized.

The sensor unit 12, 14 has a color coding field 12a, 14a which has a color. This field can also be used for naming or branding.

Moreover, each sensor unit 12 or 14 has its own electronic identification circuit 12b or 14b.

With the electronic identification circuit 12b or 14b the sensor unit 12, 14 can identify a counterelement.

As counterelement for each electronic identification circuit 12b or 14b, there is an individualized resistor R1, R2 in each sensor holder 16, 18 as passive element.

Due to the fact that the sensor holders 16, 18 have individualized counterelements, it is not necessary that the sensor units are individualized,

To the contrary, in the shown embodiment the sensor units are completely identical as no strict correspondence between sensor units 12, 14 and sensor holders 16, 18 is needed.

Moreover, only the sensor holders 16, 18 comprise an identification of the limb, where they should be placed on and also an identification of the side of the body, to which they belong to and on which they should be placed.

The sensor holders 16, 18 are embodied such that they comprise wearable elastic straps 16a, 18a.

The wearable straps 16a, 18a comprise each at least one length adjustment element 16b, 18b, which is here a clamp 16b, 18b for length adjustment of the straps 16a, 18a.

With wearable straps 16a, 18a the sensor holders 16, 18 may be placed on the body or a limb of the patient.

On each elastic strap 16a, 18a a sensor unit attachment element 16c, 18c is provided, which serves as a docking element or docking station for one of sensor units 12, 14. Each sensor unit attachment element 16c, 18c has a form-fit or snap-fit for the sensor units 12, 14.

Here the form-fit and snap-fit is realized by means of elastic holding walls 16d, 18d and a recess 16e, 18e for the L-form protrusion 12c, 14c extending out of the main body 12d, 14d of the sensor unit 12 or 14.

Furthermore, there are snap-fit fingers 16f, 18f in the walls 16d, 18d, which are protruding out of the walls 16d, 18d and are arranged such that they realize a snap-fit contact with a sensor unit 12,14, once a sensor unit 12, 14 is inserted into the sensor unit attachment element 16c, 18c.

The sensor unit attachment elements 16c, 18c are attached to wearable straps 16a, 18a.

The sensor station 22 comprises a ON-OFF-Button 22a.

Furthermore, the sensor station 22 comprises in each of its slots 22e an attachment communication interface 22b.

Also, there is a communication line 22c for connection with the main unit 20, which is here represented by a cable 22c.

Additionally, there is a Bluetooth link 22d as shown in **Fig. 3****.**

Further (wireless) Bluetooth links B1, B2, B3, B4, B5 are established between each sensor unit 12, 14 and further sensor units 32, 34, 36 on the one side and the main unit 20 on the other side/in the center of the system 10.

All sensor units 12, 14, 32, 34, 36 are completely identical.

It would also be possible, to have one Bluetooth connection between one sensor master unit and the main unit and connect the other sensor units with the sensor master unit.

All sensors units 12, 14, 32, 34, 36 are each equipped with at least one inertial sensor.

Alternatively or additionally, as sensor may be used at least one of the sensor types gyroscope, acceleration sensor, piezo sensor, magnetic sensor, optical sensor or the like.

The functionality can be described as follows:
The sensor units 12, 14, 32, 34, 36 are each configured such that depending on the correct placement of the sensor unit 12, 14, 32, 34, 36 to the sensor holder 16, 18 a communication link L1, L2 with the communication interface 24, 26 (of the sensor units) and the counter communication interface 28, 30 (of the sensor holder) is established.

Then, it can be inferred by the sensor unit 12, 14, whether it is attached to the sensor holder 16, 18 or not.

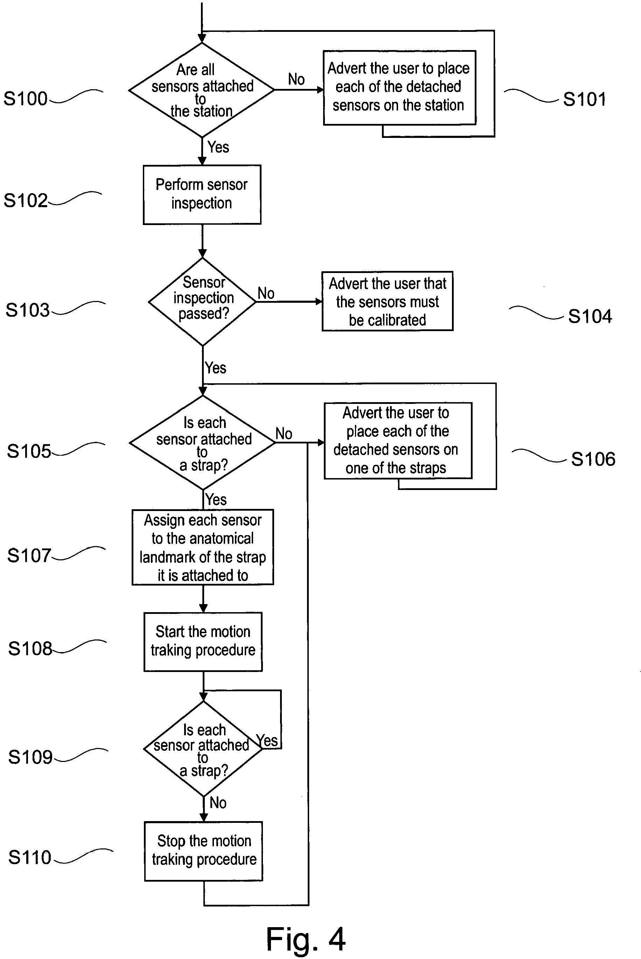

As described below in connection with **Fig. 4****,** it is not necessary to have the sensor units and the sensor holders assigned to each other before the procedure.

The inspection procedure is a procedure where the sensor units are kind of "quality controlled" from the point of view that the measured values/signals provided by the sensor units are correct.

The main unit 20 is configured such that motion data as well as the information regarding the attachment of the sensor unit 12, 14 to the sensor holder 16, 18 can be tracked and/or processed.

The main unit 20 processes the motion data as well as the information regarding the attachment of each sensor unit 12, 14, 32, 34, 36 and provides feedback to the user via the display of a tablet device, here forming the main unit 20.

Sensor units 12, 14, 32, 34, 36 (also called motion sensors) are placed in anatomical landmarks, they collect the user's motion data as well as information regarding which component they are attached to and send it to the main unit 20 through a wireless communication protocol (here the sensor units 12, 14, 32, 34, 36 are embodied as devices comprising a microcontroller, Bluetooth connectivity, inertial sensors and the attachment communication interface).

The sensor holders 16, 18 with the wearable elastic straps 16a, 18a securely attach each sensor unit 12, 14, 32, 34, 36 to the corresponding anatomical landmark (of a patient).

Each sensor holder 16, 18 is equipped with a counter communication interface 28, 30 (also called attachment communication interface 28, 30).

The sensor station 22 holds the sensor units 12, 14, 32, 34, 36 for charging and inspection purposes. Equipped with the attachment communication interface 22b, this component communicates with the main unit 20 through the cable/ communication line 22c and/or the wireless communication protocol, here the additional Bluetooth link 22d.

This interface, i.e. the attachment communication interface 22b of the sensor station 22 or the counter communication interface 28, 30 of the sensor holder 16, 18 provides the advantage that the sensor units 12, 14, 32, 34, 36 have the capability of inferring to which piece of hardware they are connect (if any).

In its simplest form, it can be embodied as a couple of terminals connected by a resistor on the sensor holder/sensor station side and an active circuit on the sensor unit side. With such configuration and by having a different resistor value for each of the straps as well as the sensor station, the microcontroller in the sensor is able to measure resistance between its terminals continuously and thus infer whether it is disconnected, connected to one of the straps or to the sensor station. By having a passive circuit on the sensor holder side, one can avoid the need of charging the straps which is obviously of interest for the sake of the user experience.

Each of the sensor holders 16, 18, as well as the sensor station 22, is equipped with a hardware interface that connects to a homologous interface in the sensors upon their attachment. This interface and the connection provide each individual sensor unit with the capability of inferring whether it is attached to a specific strap, attached to the base station or detached. As a result the system is able to:
- Make sure the sensor units are correctly attached to the sensor holders and advert the user if they are not before the motion tracking procedure begins.
- Advert the user when a sensor detaches from its strap during the motion tracking procedure.
- Make sure the sensor inspection procedure is valid by requiring the user to have the sensors correctly attached to the station.
- Ensure a proper charging procedure of the sensors by requiring the user to have the sensors correctly attached to the station.
- Allow the user to attach each sensor to a random strap since the system is now capable of performing the match between each sensor and the strap without having to rely on a predefined configuration the user would have to comply with (as is the case of the color codes).

These features have an advantageous and beneficial impact on the user experience as the system is now able to guide the user through each step by providing relevant and adaptive feedback whilst ensuring proper functioning.

Generally speaking the method of using the system 10 has at least the following steps:
- the sensor unit(s) 12, 14, 32, 34, 36 is/are placed in the sensor holder(s) 16, 18;
- a communication link L1, L2 between the sensor unit 12, 14 and the sensor holder 16, 18 is established; and
- based on the communication link L1, L2 and the information exchanged via this communication link L1, L2 it is inferred by the sensor unit 12, 14, whether it is attached to the sensor holder 16, 18 or not.

With the established communication link L1, L2 an individualized element of the sensor holder 16, 18, here the resistors R1, R2, is/are checked and identified by means of the sensor unit 12, 14, 32, 34, 36.

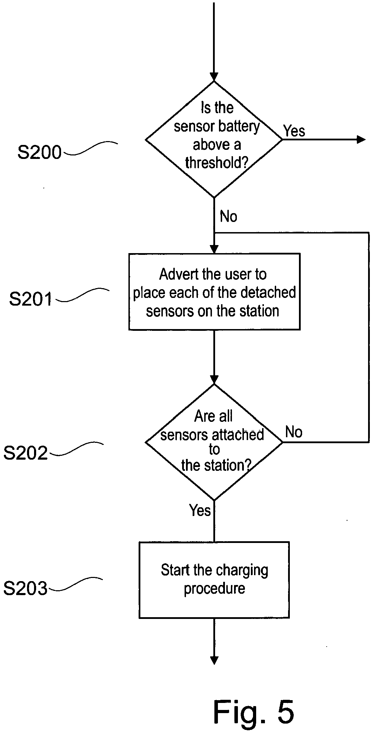

Further details are explained in connection with **Fig. 4** and **Fig. 5****.**

**Fig. 4** shows a flow chart of the system setup procedure (also including a sensor unit inspection routine)

This routine comprises the necessary steps a user must perform in the shown embodiment of the system to start tracking its motion with the system 10. Similar setups and routines are generally possible.

Here, at least one or more or all of the sensor units 12, 14, 32, 34, 36 is/are attached to the sensor station 22, i.e. the at least one sensor unit 12, 14, 32, 34, 36 is put in one of the slots 22e.

In step S100 it is checked, whether all sensor units 12, 14, 32, 34, 36 (here all, it might in an alternative embodiment be also possible that for the check only one or several sensor units or all sensor units must be placed into the sensor station 22) are attached to the sensor station 22.

If this is not the case, the procedure continues in step S101, i.e. the user is invited to place each of the detached sensors into the slots 22e of the sensor station 22. Then, it is continued with step S100 again.

If all sensor units 12, 14, 32, 34, 36 are then attached to the sensor station 22, then in step S102 the sensor unit inspection is performed.

After the sensor inspection of step S102 a decision is taken in step S103, whether the sensor inspection is passed or not.

If the sensor inspection is not passed, then in step S104 the user is invited to calibrate the sensor units 12, 14, 32, 34, 36.

If the sensor inspection is passed, then in step S105 it is checked, whether each sensor unit 12, 14, 32, 34, 36 is attached to one sensor holder 16, 18.

This is checked as described above by means of the sensor communication interface 24, 26 and the passive counter communication interface 28, 30 of the sensor holder 16, 18.

If the check of step S105 reveals that not all sensor units 12, 14, 32, 34, 36 are correctly placed, the user is invited in step S106 to place each of the detached sensors units 12, 14, 32, 34, 36 to one sensor holder, 16, 18 (one sensor unit to one sensor holder).

Steps S101 ("Advert the user to place each of the detached sensors on the station"), S104 ("Advert the user that the sensors must be calibrated") and S106 ("Advert the user to place each of the detached sensors on one of the straps") can involve the step of displaying such a message by means of the display on the main unit 20.

If the system 10 comes in check S105 to the result that each sensor is attached to a sensor holder 16, 18, then in step S107 each sensor unit 12, 14, 32, 34, 36 is assigned to the anatomical landmark of the sensor holder 16, 18 it is attached to.

After that, in step S108 the motion tracking procedure is started.

During this procedure, there is a check routine in step S109, which is following step S108. Here, it is continuously (or alternatively intermittently) checked, whether each sensor unit 12, 14, 32, 34, 36 is still attached to a sensor holder 16, 18.

In step S110, the motion tracking procedure is stopped, when in step S109 it is found that one or more of the sensor units 12, 14, 32, 34, 36 are no longer attached to the respective sensor holder 16, 18.

If so, it is switched back to step S106.

Then the procedure may continue from there on.

**Fig. 5** shows a flow chart of the sensor unit charging procedure according to the present invention (also sensor unit charging routine).

In starting step S200 it is checked, whether the battery status of the battery of the sensor unit 12, 14, 32, 34, 36 is above a threshold.

Such a threshold may be a preset value, which may be chosen by the manufacturer and is orientated on a charging level, which ensures proper functioning plus safety surplus.

If this check reveals that the charging status of the battery of the sensor unit 12, 14, 32, 34, 36 is above the preset threshold, then the sensor unit charging routine is ended.

If the check reveals that the charging status of the battery of the sensor unit 12, 14, 32, 34, 36 is below the preset threshold, then the user is invited in step S201 ("Advert the user to place each of the detached sensors on the station") to put the sensor unit(s) 12, 14, 32, 34, 36 into the slots 22e of the sensor station 22.

This message can be displayed via the display of the main unit 20.

It is possible that the recharging of all sensor units 12, 14, 32, 34, 36 is required at the same time (as shown in Fig. 5). This requirement is based on the assumption that all sensor units 12, 14, 32, 34, 36 will in service at the same time and will more or less consume the same amount of power during this service period. If at least one sensor unit 12, 14, 32, 34, 36 is detected with a battery level below the threshold, then recharging for all sensor units 12, 14, 32, 34, 36 is required as all others will follow soon with a recharging requirement.

It is however, also possible, that only the recharging of those sensor units 12, 14, 32, 34, 36 is required, which need a recharging.

In step S202 it is checked, whether all sensors are attached to the sensor station 22.

If this is not the case, then the procedure continues at step S201.

If it is detected that all sensor units 12, 14, 32, 34, 36 are attached to the sensor station 22, then the charging procedure is started in step S203.

The start can be done automatically. It is also possible that a manual start must be done, e.g. by pushing the ON-OFF-Button 22a.

Note that the example control and estimation routines included herein can be used with various system configurations. The control methods and routines disclosed herein may be stored as executable instructions in non-transitory memory and may be carried out by a control unit e.g. as a part of the main unit 20 in combination with the various sensors, actuators, and other system hardware. The specific routines described herein may represent one or more of any number of processing strategies such as event-driven, interrupt-driven, multi-tasking, multi-threading, and the like. As such, various actions, operations, and/or functions illustrated may be performed in the sequence illustrated, in parallel, or in some cases omitted. Likewise, the order of processing is not necessarily required to achieve the features and advantages of the example embodiments described herein, but is provided for ease of illustration and description. One or more of the illustrated actions, operations and/or functions may be repeatedly performed depending on the particular strategy being used. Further, the described actions, operations and/or functions may graphically represent code to be programmed into non-transitory memory of the computer readable storage medium in the control unit, where the described actions are carried out by executing the instructions in a system including the various hardware components in combination with the electronic control unit.

### References

- 10: system for tracking human motion
- 12: sensor unit
- 12a: color coding field
- 12b: electronic identification circuit
- 12c: L-form protrusion
- 12d: main body
- 14: sensor unit
- 14a: color coding field
- 14b: electronic identification circuit
- 14c: L-form protrusion
- 14d: main body
- 16: sensor holder
- 16a: strap
- 16b: clamp
- 16c: sensor unit attachment element
- 16d: wall
- 16e: recess
- 16f: snap fit finger
- 18: sensor holder
- 18a: strap
- 18b: clamp
- 18c: sensor unit attachment element
- 18d: wall
- 18e: recess
- 18f: snap fit finger
- 20: main unit
- 22: sensor station
- 22a: ON-OFF-Button
- 22b: attachment communication interface
- 22c: communication line
- 22d: Bluetooth link
- 22e: slot
- 24: sensor communication interface
- 26: sensor communication interface
- 28: counter communication interface
- 30: counter communication interface
- 32: sensor unit
- 34: sensor unit
- 36: sensor unit

- L1: communication link
- L2: communication link

- S100: step of sensor unit inspection routine
- S101: step of sensor unit inspection routine
- S102: step of sensor unit inspection routine
- S103: step of sensor unit inspection routine
- S104: step of sensor unit inspection routine
- S105: step of sensor unit inspection routine
- S106: step of sensor unit inspection routine
- S107: step of sensor unit inspection routine
- S108: step of sensor unit inspection routine
- S109: step of sensor unit inspection routine
- S110: step of sensor unit inspection routine

- S200: step of sensor unit charging routine
- S201: step of sensor unit charging routine
- S202: step of sensor unit charging routine
- S203: step of sensor unit charging routine

- R1: resistor
- R2: resistor

## Claims

1. A system (10) for tracking human motion comprising
- at least one sensor unit (12, 14),
- at least one sensor holder (16, 18),
wherein the sensor unit (12, 14) comprises a sensor communication interface (24, 26) and wherein the sensor holder (16, 18) comprises a counter communication interface (28, 30), wherein the sensor unit (12, 14) is configured such that depending on the correct placement of the sensor unit (12, 14) to the sensor holder (16, 18) a communication link (L1, L2) with the sensor communication interface (24, 26) and the counter communication interface (28, 30) is established and it can be inferred by the sensor unit (12, 14), whether it is attached to the sensor holder (16, 18) or not.

2. The system (10) according to claim 1, **characterized in that** the sensor holder (16, 18) is a wearable, especially wherein the sensor holder (16, 18) is a wearable strap (16a, 18a).

3. The system (10) according to claim 1 or claim 2, **characterized in that** the sensor unit (12, 14) comprises at least one of an inertial sensor, gyroscope, acceleration sensor, piezo sensor, magnetic sensor, optical sensor or the like.

4. The system (10) according to one of the preceding claims, **characterized in that** the system (10) further comprises a main unit (20), wherein the main unit (20) configured such that motion data as well as the information regarding the attachment of the sensor unit (12, 14) to the sensor holder (16, 18) can be tracked and/or processed.

5. The system (10) according to one of the preceding claims, **characterized in that** the system (10) further comprises a sensor station (22), which is configured and arranged for re-charging of the sensor unit (12, 14).

6. The system (10) according to claim 5, **characterized in that** the sensor station (22) comprises a plurality of receiving slots (22e) for a sensor unit (12, 14, 32, 34, 36).

7. The system (10) according to one of the preceding claims, **characterized in that** the system (10) comprises a plurality of sensor units (12, 14, 32, 34, 36) and/or a plurality of sensor holders (16, 18).

8. The system (10) according to claim 7, **characterized in that** all sensor units (12, 14, 32, 34, 36) are identical and/or all sensor holders (16, 18) are individualized and different from each other.

9. The system (10) according to one of the preceding claims, **characterized in that** the counter communication interface (28, 30) comprises at least one passive element.

10. The system (10) according to claim 9, **characterized in that** the passive element is or comprises a resistor (R1, R2).

11. The system (10) according to claim 10, **characterized in that** the resistor (R1, R2) has a specific resistance, which is to a specific sensor holder (16, 18).

12. The system (10) according to claim 11, **characterized in that** a plurality of sensor holders (16, 18) is provided and each sensor holder (16, 18) has its own specific resistor (R1, R2) with its own specific resistance, thereby identifying the sensor holder (16, 18).

13. A method for sensor unit placement verification for a system (10) for tracking human motion, wherein the system (10) comprises
- at least one sensor unit (12, 14),
- at least one sensor holder (16, 18),
further comprising the steps:
- the sensor unit (12, 14, 32, 34, 36) is placed in the sensor holder (16, 18);
- a communication link (L1, L2) between the sensor unit (12, 14) and the sensor holder (16, 18) is established; and
- and based on the communication link (L1, L2) and the information exchanged via this communication link (L1, L2) it is inferred by the sensor unit (12, 14), whether it is attached to the sensor holder (16, 18) or not.

14. The method of claim 13, **characterized in that** with the established communication link (L1, L2) an individualized element of the sensor holder (16, 18) is checked and identified by means of the sensor unit (12, 14, 32, 34, 36).

15. The method of claim 13 or claim 14, **characterized in that** the system (10) for tracking human motion is a system (10) for tracking human motion according to one of claims 1 to 13.
